Europäisches Patentamt

European Patent Office

Office européen des brevets

(11) Publication number: **0 389 700**
**A1**

## EUROPEAN PATENT APPLICATION

(21) Application number: 89303098.1

(51) Int. Cl.5 **A61K 9/48**

(22) Date of filing: 29.03.89

The title of the invention has been amended (Guidelines for Examination in the EPO, A-III, 7.3).

(43) Date of publication of application:
**03.10.90 Bulletin 90/40**

(84) Designated Contracting States:
**DE FR GB**

(71) Applicant: FUJI CAPSULE KABUSHIKI KAISHA (also trading as FUJI CAPSULE CO., LTD.)
1035 Onakazato
Fujinomiya-shi Shizuoka(JP)

(72) Inventor: Goto, Hiroshi
597-2 Miyahara
Fujinomiya-shi Shizuoka(JP)
Inventor: Kondo, Takashi
3-10-21 Irie
Shimizu-shi Shizuoka(JP)

(74) Representative: Spencer, Graham Easdale et al
A.A. Thornton & CO Northumberland House
303-306, High Holborn
London WC1V 7LE(GB)

(54) Soft agar capsules.

(57) The present invention relates to a soft capsule and a globular material which are applied to pharmaceuticals, chemicals, cosmetics, foodstuffs, miscellaneous goods and the like, and particularly to a soft capsule and a globular material prepared from agar-agar as the base material thereof and having different characteristics from those of conventional soft capsules and globular materials.

EP 0 389 700 A1

## SOFT CAPSULE AND GLOBULAR MATERIAL

Field of the Invention and Related Art Statement

The present invention relates to a soft capsule and a globular material (hereinafter referred to as "soft capsule and the like") which are applied to pharmaceuticals, chemicals, cosmetics, foodstuffs, miscellaneous goods and the like, and particularly to a soft capsule and the like prepared from agar-agar as the base material thereof and having different characteristics from those of conventional soft capsules and the like.

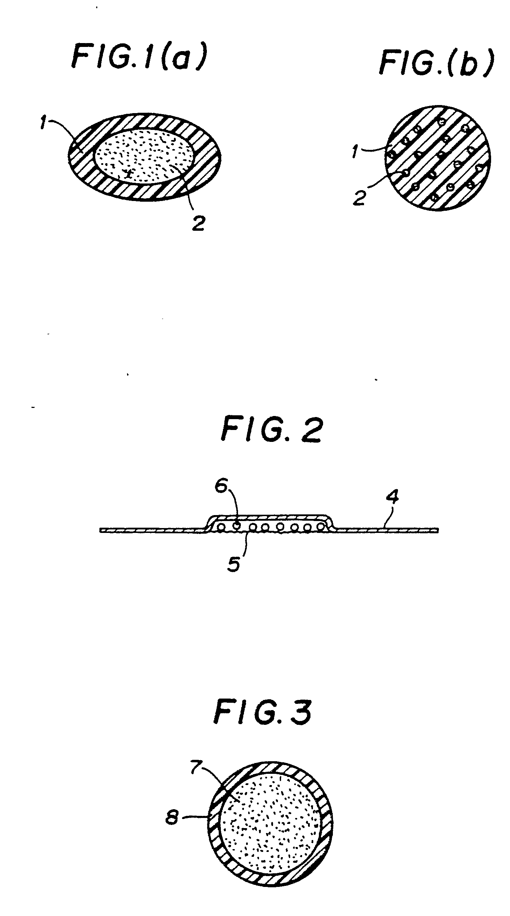

Heretofore, a capsule made from gelatin as the base material has been used for a wide variety of applications. For example, a typical soft capsule has a shape as shown in Fig. 3 wherein reference numeral 7 designates liquid contents such as liver oil, vitamin E or the like, and 8 a film made from gelatin as the base material, respectively. Furthermore, uses of such soft capsule are developing into ones other than pharmaceuticals, i.e. cosmetic uses such as bath oil, perfume and the like; health food uses such as squalene, evening primrose oil and the like; ordinary foodstuff uses such as seasoning and the like; room aromatics; projectiles for toy pistol; and the like.

Meanwhile almost all the film base materials for soft capsule used heretofore are gelatines, while alginates and carrageenans have been very scarcely used.

A reason why gelatin is suitable for a base material of soft capsule is in that there was no material other than gelatin which meets such requirements that it satisfies the quality and function required for the base material of soft capsule film, it is inexpensive, and its access to the raw material is easy and stable.

When enumerated, physical properties required for a base material of soft capsule are as follows:

(1) Being edible and safe.

(2) Forming instantaneously a film upon thermally reversible, chemical or the like reaction in respect of sol-gel.

(3) Having a prescribed film strength and stretchability.

(4) Exhibiting little reactivity with respect to the contents in a capsule and being stable with age.

(5) Having heat bond performance (heat sealability) (which is necessary for only a stamping method among methods for manufacturing soft capsules).

In view of the above, gelatin satisfies all the physical properties required as enumerated above.

On the other hand, soft capsules according to the prior art have the following restrictions, because each film of them is principally made from gelatin as the base material.

(1) Since gelatin is an animal protein, a soft capsule made of a gelatin base material is unsuitable for peoples who cannot take animal proteins in view of reasons for vegetarianism and religious problems.

(2) Since gelatin is a protein, it is reactive in the case when reducing materials or aldehydes are contained in the contents of a capsule so that such capsule is unstable.

(3) Gelatin deteriorates under the exposition of direct rays of the sun during its preservation.

(4) Gelatin softens and changes in its shape at high temperatures (about 40°C or more) under high humidity, so that the films of capsules adhere to each other.

For instance, commodities in which soft capsule made from gelatin as its base material is used cannot sold in automatic vending machines which are usually exposed to various conditions from the viewpoint of preservation.

In these circumstances, soft capsule made from a gelatin base material has been restricted to its applications in respect of a certain field.

Summary of the Invention

The present invention has been made in view of the above and contemplates to provide a novel soft capsule and globular material which are made from an agar-agar base material as well as the applications therefor.

All of the above-mentioned problems in conventional soft capsules are derived from the essence of gelatin. Accordingly, various natural edible polymeric materials other than gelatin have been screened as a film base material for soft capsule. As a result, it has been found that agar-agar satisfies substantially all the above-mentioned necessary conditions as a film base material, but it has not beat bond performance so that agar-agar cannot be used as a film base material for soft capsule according to a stamping method. It has been, however, discovered that agar-agar can be practically used as the film base material for soft capsule in accordance with the undermentioned drip method by only modifying somewhat conventional methods in respect of heat conrol, a water retention agent, a filler and the like.

Furthermore, a novel soft capsule and the like exhibit particular functions clearly different from those of a soft capsule made from a gelatin base material in water insolubility, heat resistance, suitable feeling in eating, a low capsule strength and the like.

Brief Description of the Drawings

Figs. 1(a) and (b) are sectional views each showing the soft capsule or a globular material according to the present invention;

Fig. 2 is a sectional view showing an adhesive tape for burn according to an embodiment of the present invention and;

Fig. 3 is a sectional view showing a gelatin soft capsule according to a prior art.

Detailed Description of the Preferred Embodiments

The soft capsule and the like according to the present invention will be described in detail hereinbelow.

For a base material of the soft capsule and the like, natural high-molecular agar-agar may be used, and particularly "Kanten" according to the Japanese Pharmacopoeia is used in pharmaceuticals, besides agarose being a fractioned and purified product of agar-agar can be also used.

As its additives, a plasticizer (water retention agent) such as glycerin, sorbitol and the like, a preservativ such as Ethyl Paraben and the like, besides a stabilizer, a colorant, a perfume, a disintegrator assistant, and a corrigent may be optionally added.

In the meantime, the soft capsule and the like according to the present embodiment are prepared in accordance with a normal drip method. The drip method is such a method for manufacturing a capsule without a seam, a so-called seamless capsule by utilizing such a tendency that a droplet makes a spherical shape by means of surface tension of the droplet.

In accordance with said dropping method, the soft capsule and the like as shown in Figs. 1(a) and (b) are obtained and both the figures show a so-called double construction in which aqueous components can be contained in a film 1 and oily components may be contained as contents 2. In this case, it is to be noted that the article shown in Fig. 1(b) is not generally called by soft capsule, so that such article is called by a globular material and is discriminated from the soft capsule.

Characteristic features of the soft capsule and the like prepared from agar-agar as the base material thereof are as follows:

(1) The soft capsule and the like prepared from an agar-agar base material dissolve hardly in water or warmed water of high temperatures, whilst gelatin capsule dissolves in warmed water of 40°C for about 10 minutes.

(2) They have high heat resistance. More specifically, gelatin transforms from gel- to sol-form at a temperature of 40°C or more, whilst agar-agar has a denaturation point of 70°C or more.

(3) The shell of a capsule is soft and has a particular feeling in eating (texture).

On the other hand, a gelatin capsule is not broken unless it is fairly bit, besides feeling is poor in eating.

(4) Since a capsule strength can be adjusted to a low level, it can be easily bit in the mouth.

Examples to which are applied the agar-agar capsule and the like having the above described construction will be enumerated hereinbelow.

[Example 1] Vegetable soft capsule agent

The soft capsule (medicine vitamin E) according to the following formulation example was prepared. In this case, it is to be noted that an amount of formulation in each component indicates that in one capsule (globular material) (This is also the same with those in the following formulation examples.).

<Formulation example>

| | | | |
|---|---|---|---|
| Capsule Liquid Contents: | Natural vitamin E | 100 | mg |
| | Soybean oil | 50 | mg |
| Capsule Film : | Agar-agar | 25 | mg |
| | Glycerin | 13 | mg |
| | Water | 2.5 | mg |

A gelatin soft capsule cannot be taken by vegetarian or peoples who have a certain religious reason, because the gelatin soft capsule is made from an animal protein. In this respect, however, all the above described formulation raw materials are derived from pure vegetables, so tht pharmaceuticals and health foods suitable for vegetarian and the like can be obtained.

[Example 2] Mouth releasing type soft capsule

The soft capsule (use for sub-lingual absorption) according to the following formulation example was prepared.

| <Formulation example> | | |
|---|---|---|
| Capsule Liquid Contents: | Nifedipine | 10 mg |
| | Macrogol 400 | 100 mg |
| Capsule Film : | Agar-Agar | 10 mg |
| | Glycerin | 10 mg |
| | Ethyl Paraben | 0.02 mg |
| | Propyl Paraben | 0.01 mg |
| | Water | 1 mg |

Nifedipine soft capsule which is used in case of a fit of heart attack usually takes internally, but there is also such a case where a capsule is bit by a patient and broken to release the liquid contents thereof in case of emergency, whereby Nifedipine is taken by means of sub-lingual absorption which produces an immediate effect on the heart attack. In this case, since a conventional gelatin soft capsule has a high breaking hardness of 10 kg or more, such capsule could not have been easily crushed with teeth. In this respect, a breaking hardness of an agar-agar soft capsule can be adjusted to as low as 5 kg or less, so that the capsule can be easily crushed with teeth or fingers of a patient and hence, such agar-agar soft capsule is the most suitable sub-lingual absorption capsule.

[Example 3] Maintaining use soft capsue

A globular material (for maintaining dissolution of ingredients) was prepared in accordance with the following formulation example.

| <Formulation example> | |
|---|---|
| Nicardipine Hydrochloride | 1 mg |
| Agar-agar | 50 mg |
| Glycerine | 50 mg |
| Water | 5 mg |

As a result of a dissolution test according to the Japanese Pharmacopoeia, the dissolution of effective ingredients was remarkably maintained in the present globular material as compared with that of a gelatin capsule containing the same amount of Nicardipine hydrochloride.

[Example 4] Soft capsule for external application

A soft capsule (for use in burn) was prepared in accordance with the following formulation example.

<Formulation example>

| Capsule Liquid Contents: | Squalane | 100 mg |
|---|---|---|
| Capsule Film : | Agar-agar | 50 mg |
| | Glycerin | 25 mg |
| | Water | 5 mg |

An adhesive tape for burn composed of an adhesive tape material 4, a gauze 5, and soft capsules 6 is prepared as shown in Fig. 2 by using said soft capsule having the above described formulation. The adhesive tape obtained is utilized in such a manner that the capsules are crushed with fingers to release the liquid contents thereof and the gauz is impregnated with the liquid contents, and thereafter the burned part is covered with the gauze thus impregnated. The present adhesive tape is simple and in which an oil can be maintained for a long period of time in comparison with a conventional medical treatment wherein an oil is directly applied on a burned part.

[Example 5] Soft capsule for water-containing cosmetics

A soft capsule (for cosmetics) was prepared in accordance with the following formulation example.

<Example of Formulation Ingredient>

Capsule Liquid Contents: Perfume, Oil, Nutritive Substance and Other Effective Ingredients
Capsule Film : Agar-agar, Glycerin
The soft capsules having the above described formulation were mixed with water-containing cosmetics such as lotion, cream, milky lotion, shampoo and the like.
While the film of a gelatin soft capsule dissolved in water with age, the film of the present soft capsule did not dissolve in, water for a long period of time, so that the present soft capsules could maintain a mixed state with the water-containing cosmetics. On one hand, globular materials each having a diameter of around 0.5 mm can be prepared from the above formulation ingredients, the resulting globular materials may be mixed with massage cream to serve a scrub cosmetic. In comparison with the present globular materials, gelatin globular materials each having a diameter of around 0.5 mm dissolved with age so that they were lack in stability.

[Example 6] Soft capsule for water-containing foodstuff

Soft capsules each containing perfumes, seasonings, nutritive substances and the like were prepared, and the resultant capsules were admixed with custard pudding, yogurt, milk or the like. In this case, the present soft capsules did not dissolve and maintained the admixed state for a long period of time, so that they were useful for stability of the perfumes, inhibiting change in taste of the foodstuff, and replenishment of nutritive substances, whilst gelatin soft capsules dissolved for several hours.

[Example 7] Soft capsule for toy pistol projectile

A soft capsule (for a toy pistol projectile) was prepared in accordance with the following formulation example.

| <Formulation example> | | |
|---|---|---|
| Capsule Liquid Contents: | Edible oil | 100 mg |
| | Oil Coloring Matter | 0.1 mg |
| Capsule Film : | Agar-agar | 20 mg |
| | Glycerin | 5 mg |
| | Water | 2 mg |

In the case when the soft capsule having the above described formulation is used as a projectile for use in a commercially available toy pistol, since the capsule is easily broken at the time of impact, an impact area colored by the coloring matter can be recognized.

As compared with commercially available paint projectiles (made of plastics), the projectile of the present example is soft, and further all the formulation ingredients thereof are edible so that the present projectile has very high safety.

[Example 8] Well-nourished soft capsule for medicated drink (medicine)

A commercially available medicated drink contains usually water-soluble vitamines and scarcely oil-soluble vitamines. If the agar-agar soft capsules containing vitamin E prepared in Example 1 are incorporated in a medicated drink, such oil-soluble vitamin comes to be possible to incorporate in the medicated drink. In addition, such ingredients having too much taste, smell and the like to directly incorporate in a medicated drink can also be mixed unrestrictedly therewith by the use of the present soft capsules.

[Example 9] Soft capsule for fishing bait

Agar-agar soft capsules in which an attractive substance to fish such as an extract of echinoid and the like is incorporated in each film portion thereof were prepared.

Since the present soft capsules did not dissolve in both seawater and fresh water, they could be used for a long period of time so that the soft capsules for fishing bait were useful as an artificial bait.

[Example 10] Soft capsule for bath lotion (chemicals)

Agar-agar soft capsules containing perfumes were prepared.

As a result of mixing the present soft capsules with a liquid type bath lotion, safety for the perfumes was elevated so that the present soft capsules were useful.

Claims

1. A soft capsule comprising agar-agar as the base material thereof.

2. A soft capsule as claimed in claim 1 wherein the film of said capsule contains aqueous ingredients, whilst the contents thereof contain oily ingredients.

3. A globular material comprising agar-agar as the base material thereof.

4. A soft capsule or globular material as claimed in claim 1, 2 or 3 wherein said capsule or globular material is composed of only vegetable ingredients.

5. A soft capsule or globular material as claimed in claim 1, 2 or 3 wherein said capsule or globular material is used for pharmaceuticals of sub-lingual absorption.

6. A soft capsule or globular material as claimed in claim 1, 2 or 3 wherein said capsule or globular material is used for continuously maintaining medicines.

7. A soft capsule as claimed in claim 1 or 2 wherein said capsule is used for toy pistol projectiles.

8. A soft capsule as claimed in claim 1 or 2 wherein said capsule is used for parmaceuticals for external application each of which is employed in such that the film thereof is broken at need to release the contents of said capsule.

9. A water-containing cosmetic comprising the soft capsules or globular materials as claimed in claim 1, 2 or 3.

10. A water-containing foodstuff comprising the soft capsules or globular materials as claimed in claim 1, 2 or 3.

11. A water-containing chemical comprising the soft capsules or globular materials as claimed in claim 1, 2 or 3.

12. A water-containing pharmaceutical comprising the soft capsules or globular materials as claimed in claim 1, 2 or 3.

## FIG.1(a)

## FIG.(b)

## FIG. 2

## FIG. 3

European Patent
Office

# EUROPEAN SEARCH REPORT

Application Number

EP  89 30 3098

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | STN INTERNATIONAL INFORMATION SERVICES DATA BASE: CHEMICAL ABSTRACTS, accession no. 111(8):63952t; & JP-A-63 238 015 (MORISHITA JINTAN CO., LTD) 04-10-1988 * Abstract * | 1-6,8, 11-12 | A 61 K   9/48 |
| Y | STN INTERNATIONAL INFORMATION SERVICES DATA BASE: CHEMICAL ABSTRACTS, accession no. 110(3):22572e; & JP-A-63 164 858 (UNICOLLOID K.K.) 08-07-1988 * Abstract * | 1-6,8-12 | |
| Y | EP-A-0 273 069  (UNI COLLOID K.K.) * Claims; page 3, line 58 * | 1-6,8-12 | |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 15-11-1989 | SCARPONI U. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document